# FASCICULE DE BREVET EUROPEEN

(11) **EP 4 216 871 B1**
(45) Date de publication et mention de la délivrance du brevet: **22.04.2026**
(21) Numéro de dépôt: 21785948.7
(22) Date de dépôt: 14.09.2021
(51) Int. Cl.: A61D 1/02

(54) **DISPOSITIF D'INJECTION D'UN FLUIDE DANS UN VOLATILE**
VORRICHTUNG ZUR FLÜSSIGKEITSEINSPRITZUNG IN EIN GEFLÜGEL
DEVICE FOR INJECTING A FLUID INTO A BIRD

(30) Priorité: 25.09.2020 EP 20306092
(43) Date de publication de la demande: 02.08.2023
(73) Titulaire: Desvac, 49124 Saint Barthelemy D'Anjou (FR)
(72) Inventeur: MAGNIAUX, Philippe, 33500 Libourne (FR)
(74) Mandataire: Plasseraud IP
(86) Numéro de dépôt international: PCT/FR2021/051570
(87) Numéro de publication internationale: WO 2022/064123

(56) Documents cités:
- WO-A1-01/03605
- CN-U- 208 958 394
- DE-A1- 102015 122 069
- FR-A1- 2 276 839
- US-A- 4 276 879

## Description

### Domaine technique

La présente invention concerne un dispositif d'injection automatique d'une substance fluide, telle qu'un vaccin, dans un volatile, notamment un poussin d'un jour d'âge.

### Technique antérieure

Afin de faire face à une demande alimentaire en croissance constante, les animaux provenant de systèmes intensifs d'élevage, ont pris une importance accrue.

Une bonne santé de ces animaux, notamment pour garantir des productions adéquates, passe par la prévention de l'apparition de problèmes sanitaires, tels que l'émergence de maladies au sein de la ferme. Cette prévention peut être effectuée, en particulier, par la vaccination des animaux.

Il est ainsi connu dans l'élevage de volailles, d'inoculer un vaccin à des poussins à un jour d'âge ou entre un (1) et vingt-et-un (21) jours.
La manipulation de ces poussins est aisée, ces derniers étant particulièrement dociles, et la finesse de leur duvet garantit une introduction aisée de l'aiguille d'injection dans la peau du poussin et par conséquent, la fiabilité de l'injection.

Pour administrer des injections en un temps, aussi court que possible, des appareils automatiques d'injection de vaccin sont typiquement mis en œuvre.

A titre d'exemple, la demande de brevet WO2018/167562A1 au nom de la présente demanderesse divulgue un appareil automatique de vaccination. D'autres appareils automatiques de vaccination sont divulgués dans FR 2 276 839 A1 et WO 01/03605 A1.

Cet appareil comprend notamment un châssis présentant une façade coudée délimitant une zone d'injection, un ensemble d'injection étant logé dans ce châssis, cet ensemble d'injection comprenant une ou plusieurs seringues et une aiguille d'injection, et un bouton d'actionnement placé dans l'angle formé par la façade coudée.

Lorsque le corps du poussin est appuyé contre ce bouton d'actionnement avec une force suffisante, un mécanisme d'entrainement déplace l'aiguille, cette dernière venant alors en projection à l'extérieur du châssis pour assurer l'administration d'une injection dans le corps du poussin.

Bien que donnant de bons résultats, cet appareil peut encore être amélioré.

Tout d'abord, chaque poussin est présenté en position couchée dans la zone d'injection de l'appareil, en vue de l'injection du vaccin.

Or, cette position non naturelle pour un poussin est non seulement source de stress pour ce dernier, mais elle requiert également la réalisation de mouvements répétitifs de l'avant-bras et du poignet correspondant de l'opérateur pour assurer une orientation adéquate du poussin.

Ainsi, on peut observer dans le temps l'apparition de troubles musculo-squelettiques (TMS) chez cet opérateur.

Un tel appareil ne permet également la présentation du poussin que d'un seul et même côté de l'appareil.

Or, une telle géométrie de l'appareil constitue nécessairement un frein au nombre de poussins pouvant être traités dans un temps donné.

Outre la fatigue accrue que peut ressentir l'opérateur suite aux gestes répétitifs accomplis pour traiter les poussins, la vaccination est rendue plus complexe pour un opérateur, lorsque le côté de l'appareil permettant l'introduction d'un poussin, ne correspond pas à la latérisation, droitière ou gauchère, de cet opérateur.

De plus, il a été observé que le positionnement du poussin dans la zone d'injection de l'appareil étant libre, il existait, compte tenu des cadences d'injection atteintes, un risque non seulement pour la santé de l'animal, mais également de blessure accidentelle pour l'opérateur.

Les pertes liées à des erreurs d'injection sont bien entendu à l'origine d'une augmentation des coûts, qui n'est pas souhaitable pour l'exploitant.

Il existe donc un besoin pressant pour un dispositif d'injection d'une substance fluide dans un animal à injecter, dont la conception originale permette de surmonter les inconvénients de l'art antérieur exposés ci-dessus.

### Objet de l'invention

La présente invention vise à pallier les inconvénients de l'art antérieur en proposant un dispositif d'injection automatique d'un liquide dans un volatile, simple dans sa conception et dans son mode opératoire, permettant une injection précise et sécurisée de ce liquide.

Un autre objet de la présente invention est un tel dispositif d'injection autorisant des cadences plus élevées de vaccination.

Encore un objet de la présente invention est un tel dispositif d'injection offrant une protection accrue de l'opérateur, tout en respectant le bien-être des volatiles.

### Exposé de l'invention

A cet effet, l'invention concerne un dispositif d'injection d'un liquide dans un volatile, ce dispositif d'injection comprenant un corps principal, lequel comporte un ensemble d'injection comprenant une aiguille d'injection et au moins une seringue, ladite aiguille étant mobile entre une position retractée et une position d'injection située dans une zone d'injection placée extérieurement à ce corps principal.
Selon l'invention, ce dispositif comprend :
- un élément de guidage placé sur une surface extérieure du corps principal, cet élément de guidage comportant un fond et des rebords latéraux s'étendant à partir de ce fond, au moins la partie supérieure du dos de l'animal à vacciner étant destinée à être appuyée contre ce fond, et déplacée, tête la première, le long d'un axe de guidage défini par ledit élément de guidage, vers ladite position d'injection,
- ledit élément de guidage étant de plus configuré pour maintenir au moins le cou dudit volatile aligné ou sensiblement aligné le long de cet axe de guidage au niveau de ladite zone d'injection,
- ladite aiguille d'injection étant agencée de sorte que lors de son déplacement entre lesdites positions retractée et d'injection, elle se déplace le long d'un axe de déplacement parallèle à la surface extérieure de ce fond, ou incliné par rapport à cette surface extérieure, cet axe de déplacement s'étendant, à l'extérieur de ce corps principal, hors d'une zone d'exclusion entourant ledit axe de guidage.

De manière avantageuse, la partie du corps du volatile dans laquelle l'injection doit être administrée, étant maintenue en position par l'élément de guidage au niveau de la zone d'injection, on obtient une précision plus grande dans l'injection réalisée et un risque moindre pour la santé du volatile.

Un tel maintien en position permet également de définir un espace de protection, dite zone d'exclusion, dans lequel l'aiguille d'injection est absente lorsqu'elle se trouve en partie à l'extérieur du corps principal, ou encore dans lequel cette aiguille ne pénètre pas durant son déplacement entre les positions retractée et d'injection, cet espace étant destiné à abriter des parties sensibles du corps du volatile, notamment sa colonne vertébrale.

Cet espace de protection n'est pas nécessairement symétrique autour de l'axe de guidage, l'homme du métier comprenant que cet espace de protection est nécessaire dans la zone où se déplace l'aiguille d'injection à l'extérieur du corps principal.

A titre d'exemple, la zone d'injection est définie comme étant l'espace s'étendant au moins autour du chemin parcouru par l'aiguille d'injection lorsqu'elle pénètre dans l'extrémité supérieure du corps de l'animal vivant jusqu'au point d'injection.

A titre purement illustratif, cet axe de guidage est un axe de symétrie de l'élément de guidage.

On entend ici par « volatile », toute espèce aviaire, telle que des oiseaux de la classe des Aves, c'est-à-dire des animaux vertébrés qui sont à plumes, ailés, bipèdes, endothermiques (à sang chaud) et aptes à pondre. Dans le contexte de la présente invention, les volatiles réfèrent plus particulièrement aux oiseaux ayant un intérêt économique et/ou agronomiques, tels que des volailles (par exemple, des poulets, des dindes, des poules, des pintades, des cailles, des perdrix et des pigeons), des oiseaux migrateurs (par exemple, des canards et des oies) et des oiseaux d'ornement (par exemple, des cygnes, des perroquets et des psittacidés).

Un tel dispositif d'injection trouve des applications notamment dans le domaine de la vaccination de poussins dans les quelques jours suivant leur naissance, soit entre un (1) et quatorze (14) jours suivant l'éclosion. Préférentiellement, la vaccination est réalisée sur des volatiles ayant entre un (1) et sept (7) jours d'âge après éclosion. Plus préférentiellement, la vaccination est effectuée sur des volatiles ayant entre un (1) et deux (2) jours d'âge après éclosion, soit 48 heures après éclosion. Encore plus préférentiellement, la vaccination est réalisée sur des volatiles à un (1) jour d'âge, soit 24 heures après éclosion

De manière avantageuse, une telle configuration du dispositif d'injection autorise un accès libre, ou encore un accès entièrement dégagé, à l'élément de guidage de sorte que l'opérateur peut faire usage de ses deux mains pour amener les volatiles en position d'injection.
La cadence de vaccination permise par ce dispositif d'injection automatique s'en trouve ainsi améliorée de manière significative.

Cet élément de guidage peut être apposé ou fixé sur la surface extérieure du corps principal, cette surface extérieure étant par ailleurs coudée pour imposer une inclinaison particulière à la tête du volatile afin de dégager l'accès à sa nuque en vue de l'introduction de l'aiguille d'injection.
L'injection est de préférence réalisée dans le bas du cou ou le haut du dos de l'animal à injecter, préférentiellement juste en dessous de la nuque.
Alternativement, cette surface extérieure peut elle-même être conformée pour définir un élément de guidage en creux dans le corps principal, cet élément de guidage présentant un fond contre lequel la partie du corps du volatile dans laquelle l'injection doit être administrée, est plaquée.

Selon un mode de réalisation de ce dispositif d'injection, la partie du fond de l'élément de guidage placée au niveau du chemin le long duquel se déplace l'aiguille entre les positions retractée et d'injection, est plane ou sensiblement plane.

Selon un autre mode de réalisation de ce dispositif d'injection, l'axe de déplacement de ladite aiguille entre lesdites positions retractée et d'injection, est incliné d'un angle compris entre ]0, 4°] par rapport à la surface extérieure dudit fond.
On s'assure ainsi que l'aiguille d'injection ne peut pas pénétrer en profondeur dans le corps de l'animal lors de l'administration de l'injection. A titre d'exemple, cet angle d'inclinaison entre l'axe de déplacement de l'aiguille et le fond est de 2 +/- 2°. Un tel angle peut être obtenu de diverses manières, par exemple en maintenant le fond fixe et en pivotant l'aiguille d'injection ou encore en conservant l'orientation de l'aiguille d'injection inchangée, et en pivotant le fond. Bien entendu, il est encore possible de pivoter l'aiguille et le fond pour obtenir un tel angle d'inclinaison de l'axe de déplacement de l'aiguille par rapport au fond de l'élément de guidage.

Selon encore un autre mode de réalisation de ce dispositif d'injection, cette zone d'exclusion est un espace s'étendant sur 2 à 4 mm autour dudit axe de guidage.
Autrement dit, dans la position d'injection, l'extrémité libre de l'aiguille d'injection est placée à une distance comprise entre 2 et 4 mm de l'axe de guidage.

A titre purement illustratif, l'élément de guidage étant conformé pour aligner, ou sensiblement aligner, au moins le rachis cervical de l'animal sur cet axe de guidage, par exemple, par un rétrécissement de cet élément de guidage maintenant son cou en position, l'aiguille est agencée de sorte que le chemin qu'elle parcourt entre les positions retractée et d'injection, est décalé latéralement d'une distance d par rapport à cet axe de guidage, et par conséquent, de la position du rachis cervical de ce volatile à injecter.
On s'assure ainsi avantageusement que l'aiguille d'injection est uniquement introduite dans des tissus mous du cou ou du haut du dos du volatile et qu'aucun contact avec la colonne vertébrale de l'animal n'est réalisé lors de l'injection.

De manière plus générale, cet élément de guidage est configuré de sorte que la colonne vertébrale du volatile est placée dans la zone d'exclusion, lorsque au moins la partie supérieure du dos de l'animal à vacciner est plaquée contre son fond et que au moins le cou de cet animal est maintenu aligné ou sensiblement aligné le long de cet axe de guidage au niveau de ladite zone d'injection.

Selon encore un autre mode de réalisation de ce dispositif d'injection, l'axe de déplacement de ladite aiguille d'injection étant contenu dans un plan parallèle au fond de l'élément de guidage, cet axe de déplacement et l'axe de guidage sont colinéaires ou cet axe de déplacement de l'aiguille diverge de cet axe de guidage.
Cet axe de déplacement de l'aiguille peut ainsi être parallèle à l'axe de guidage en étant espacé, ou décalé, de ce dernier pour ne pas traverser ou être dans la zone d'exclusion.
Alternativement, cet axe de déplacement de l'aiguille d'injection peut être placé de biais par rapport à l'axe de guidage mais en s'écartant, ou encore en divergeant, de ce dernier, en allant vers l'extérieur du corps principal de sorte que l'aiguille soit bien hors de la zone d'exclusion lorsqu'elle se situe dans la zone d'injection.

Selon encore un autre mode de réalisation de ce dispositif d'injection, ce dispositif est configuré de sorte que son corps principal étant placé sur une surface horizontale plane, le volatile est injecté en position verticale, ou sensiblement verticale, c'est-à-dire en position debout.

La tête du volatile étant dirigée vers la partie supérieure du corps principal, c'est-à-dire le volatile étant déplacé dans l'élément de guidage tête la première, le poids du corps de ce volatile assure son positionnement vertical et une présentation de ce dernier au point d'injection en position debout.
La sortie de l'aiguille d'injection peut dès lors se faire verticalement du haut vers le bas.

On constate également avec un tel mode de réalisation que les risques pour l'opérateur de développer une maladie chronique liée à la manipulation des poussins sont considérablement limités

Selon encore un autre mode de réalisation de ce dispositif d'injection, au droit de la position d'injection, la distance séparant l'extrémité libre de cette aiguille d'injection de la surface extérieure du fond détermine une injection sous-cutanée dans le cou de ce volatile.
De préférence, cette distance est typiquement comprise entre trois (3) et sept (7) mm.

On s'assure ainsi que l'aiguille ne peut pas pénétrer en profondeur dans le corps de l'animal lors de l'administration de l'injection.
Des tests réalisés sur des poussins d'un jour d'âge ont démontré la précision de l'injection réalisée avec le dispositif de l'invention, notamment l'absence de sang lors du retrait de l'aiguille d'injection ("bloody neck")

A titre purement illustratif, le déplacement de l'aiguille entre les positions retractée et d'injection est de l'ordre de 23 à 27 mm.

Selon encore un autre mode de réalisation de ce dispositif d'injection, une partie du corps de cet élément de guidage est configurée pour former une barrière de protection placée au moins de part et d'autre du chemin le long duquel se déplace ladite aiguille entre les positions retractée et d'injection.
De manière avantageuse, les rebords de l'élément de guidage sont ainsi configurés pour définir une telle barrière de protection, ces rebords formant de plus des rails de guidage latéraux.
La sécurité de l'opérateur est ainsi sensiblement renforcée. Tout risque de piqûre accidentelle de l'opérateur est supprimé du fait de l'élévation de la barrière de protection proposée.

Selon encore un autre mode de réalisation de ce dispositif d'injection, cet élément de guidage comporte une première partie de corps de forme évasée et une deuxième partie de corps formant un rétrécissement, la première partie de corps étant agencée pour assurer un guidage de la partie supérieure du corps du volatile dans laquelle l'injection doit être administrée, vers ce rétrécissement.
Ce rétrécissement est positionné avantageusement au niveau de la zone d'injection.
La première partie de corps de cet élément de guidage est donc destinée à guider, de préférence de manière progressive, la partie haute du corps du volatile vers la deuxième partie de corps de l'élément de guidage formant un rétrécissement. Une fois reçue dans ce rétrécissement, la partie haute du corps du volatile, notamment son cou, se retrouve alors calée pour recevoir l'aiguille d'injection.
Un tel guidage du haut du corps de l'animal à injecter autorise également une vaccination à l'aveugle des volatiles.
A titre d'exemple, l'opérateur peut être placé en arrière du dispositif d'injection ou en toute autre position autour de cet appareil, c'est-à-dire sur les 360° autour de ce dernier.
De manière avantageuse, la partie de corps de l'élément de guidage formant un rétrécissement, est configurée pour être ajustable notamment dans sa dimension transversale, ou encore en largeur, pour s'adapter à la taille du volatile.

Selon encore un autre mode de réalisation de ce dispositif d'injection, le corps principal comporte des évidements disposés latéralement à cet élément de guidage pour faciliter l'introduction par l'opérateur du volatile à injecter dans cet élément de guidage.

Selon encore un autre mode de réalisation de ce dispositif d'injection, ce corps principal comporte au moins un capteur de force, préférentiellement, deux capteurs de force.
Le déplacement de l'aiguille est commandé uniquement par le déclenchement de ce capteur, ou de ces capteurs simultanément.
De préférence, ces capteurs de force sont avantageusement agencés par rapport à l'élément de guidage pour assurer un alignement, ou sensiblement un alignement, de la partie de la colonne vertébrale du volatile située au moins en cette position d'injection avec la surface extérieure de ce fond, lorsque ledit volatile est plaqué contre l'élément de guidage et en appui contre lesdits capteurs de force.

Selon encore un autre mode de réalisation de ce dispositif d'injection, l'extrémité inférieure de l'élément de guidage est en communication avec un toboggan assurant une évacuation du volatile après administration de l'injection.
Une fois l'injection administrée, l'opérateur peut simplement lâcher le volatile, lequel peut tomber dans un panier de stockage ou être récupéré par le toboggan pour être évacué vers une zone de stockage ou une autre zone de travail.
De préférence, ce toboggan est orientable sur 180°. Cette libre orientation du toboggan peut être réalisée, par exemple, autour d'un axe de rotation parallèle à l'axe de guidage défini par l'élément de guidage.

Selon encore un autre mode de réalisation de ce dispositif d'injection, chaque ensemble d'injection comprenant également un actionneur linéaire, cet actionneur linéaire est un actionneur hydraulique, pneumatique ou électrique.

Selon encore un autre mode de réalisation de ce dispositif d'injection, l'aiguille est déportée par rapport à la seringue ou aux seringues.
L'ensemble formé par un collecteur et le support d'aiguille est ainsi mobile et indépendant de l'unité constituée par le vérin et l'amortisseur. Cet ensemble est facilement démontable pour des opérations de maintenance ou de changement d'aiguille d'injection.
Chaque aiguille d'injection n'étant ainsi pas placée dans le prolongement de sa seringue, un dispositif d'injection plus compact est dès lors obtenu.

Selon encore un autre mode de réalisation de ce dispositif d'injection, ce dispositif comporte deux seringues et une aiguille d'injection.
De manière avantageuse, l'actionnement d'un bouton de commande placé sur le corps principal du dispositif d'injection permet de passer d'une simple à une double injection (activation d'un ou des deux vérins).

Selon encore un autre mode de réalisation de ce dispositif d'injection, la ou les seringues sont agencées dans le corps principal de manière à assurer l'évacuation de bulles d'air qui pourraient se former dans la seringue correspondante.

Selon encore un autre mode de réalisation de ce dispositif d'injection, les angles des pièces constitutives de ce dispositif d'injection avec lesquelles le volatile à injecter et/ou l'opérateur sont susceptibles d'entrer en contact sont arrondis.
On empêche ainsi toute blessure accidentelle de l'animal à injecter ainsi que de l'opérateur.

Par ailleurs, le nettoyage du dispositif d'injection est plus aisé.

Selon encore un autre mode de réalisation de ce dispositif d'injection, l'extrémité supérieure dudit corps principal présente un coude pour imposer une inclinaison particulière à la tête du volatile, ce coude comportant un ou plusieurs éléments de maintien configurés pour bloquer en position la tête du volatile à vacciner.
On s'assure ainsi d'un calage en position de la tête du volatile qui ne peut dès lors plus tourner celle-ci. C'est ainsi toute la partie supérieure du corps de l'animal vivant à vacciner qui est maintenue aligné ou sensiblement aligné le long de l'axe de guidage.

### Brève description des dessins

D'autres avantages, buts et caractéristiques particulières de la présente invention ressortiront de la description qui va suivre, faite, dans un but explicatif et nullement limitatif, en regard des dessins annexés, dans lesquels:
**Fig. 1**

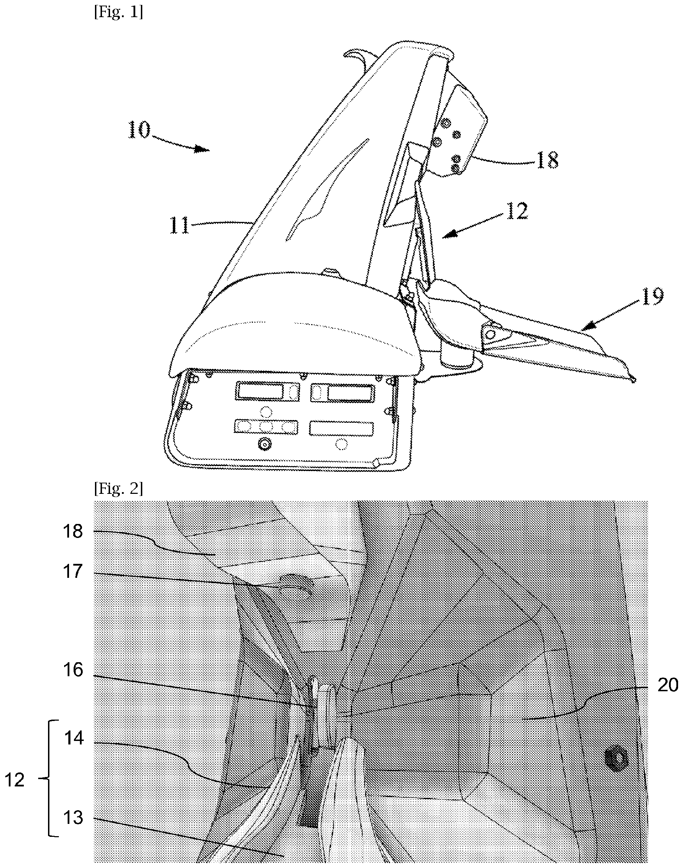

   [Fig. 1] est une vue de profil d'un dispositif d'injection automatique d'une substance liquide dans un volatile selon un mode de réalisation particulier de la présente invention ;
**Fig. 2**
   [Fig. 2] est une vue partielle et en perspective du dispositif d'injection de la Fig. 1 montrant l'agencement de l'extrémité supérieure de l'élément de guidage en relation avec les boutons d'actionnement ;
**Fig. 3**

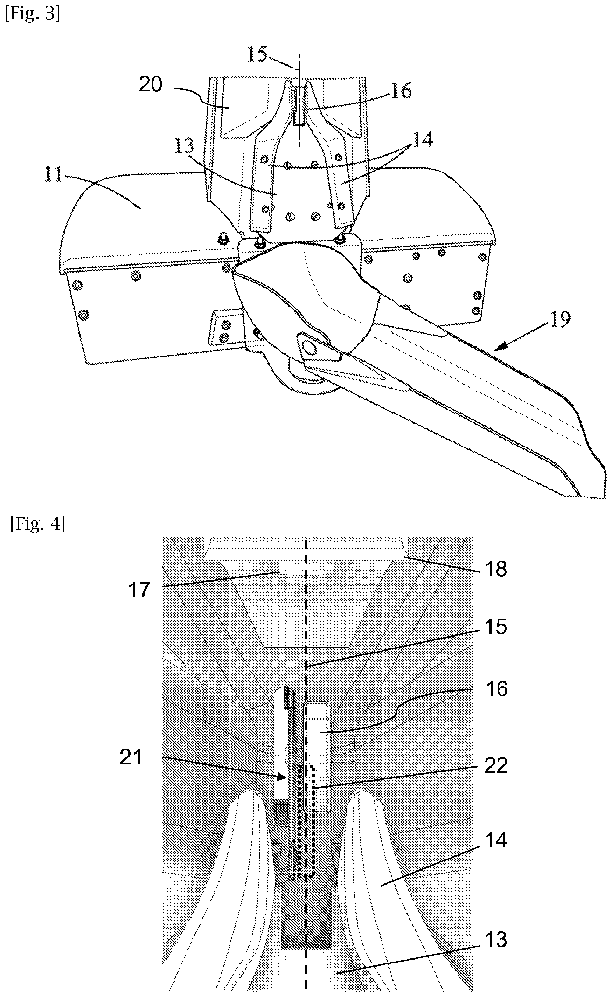

   [Fig. 3] est une vue partielle et en perspective du dispositif d'injection de la Fig. 1 montrant la partie inférieure de ce dernier ;
**Fig. 4**
   [Fig. 4] est une vue partielle et de face du dispositif d'injection de la Fig. 1 montrant l'agencement de l'extrémité supérieure de l'élément de guidage en relation avec les boutons d'actionnement ;
**Fig. 5**

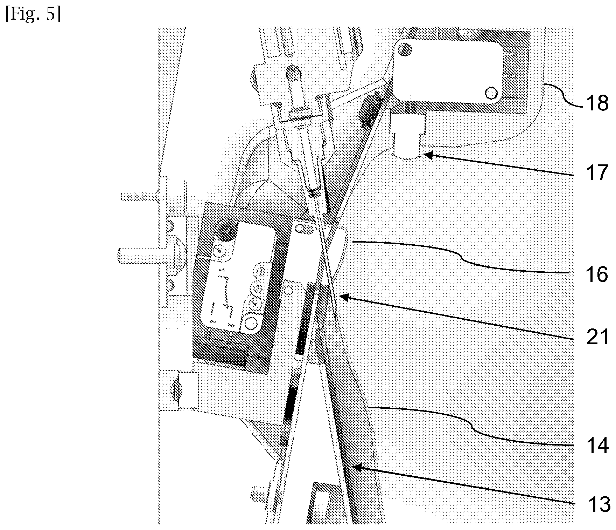

   [Fig. 5] est une vue en coupe longitudinale du dispositif d'injection de la Fig. 1 ;

### Description d'un mode de réalisation

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

Tout d'abord, on note que les figures ne sont pas à l'échelle.

Les Figures 1 à 5 illustrent de manière schématique un dispositif d'injection d'une substance liquide dans un volatile selon un mode de réalisation particulier de la présente invention.

Ce dispositif d'injection 10 comporte un châssis 11 à l'intérieur duquel est placé un ensemble d'injection. Cet ensemble d'injection comporte, de manière connue, une ou deux seringues et une aiguille d'injection.

Chaque seringue est alimentée en substance liquide par un réservoir (non représenté), tel qu'une poche souple, placé à l'extérieur du châssis 11. Ce réservoir qui détermine un volume de stockage dans lequel est reçu cette substance liquide, est supporté par un porte poche (non représenté), éventuellement télescopique. Il est ici relié au volume intérieur de chaque seringue par un conduit souple tel qu'un tuyau souple.
Chaque seringue est de préférence orientée en position verticale, ou sensiblement verticale, avec une évacuation de la substance liquide vers le haut, pour permettre l'évacuation d'éventuelles bulles d'air qui viendraient à se former.

La surface extérieure de ce châssis 11 comporte un support 12 pour recevoir le volatile.

Le corps du châssis 11 présente de chaque côté de ce support 12 des évidements, ou encore des dégagements, 20 pour faciliter le passage des pouces de l'opérateur sans que ce dernier ne soit contraint à plier ses poignets. L'introduction d'un poussin dans le support 12 est ainsi rendue plus aisée.

Ce support 12 comporte un fond 13 et des rebords 14 s'étendant à partir de ce fond vers l'extérieur. Ces rebords 14 encadrent latéralement ce fond 13 en formant saillie de ce dernier.

Ce support 12 comporte une portion de corps de forme évasée dans sa partie inférieure et une portion de corps en forme de rétrécissement dans sa partie supérieure, la portion de forme évasée débouchant dans le rétrécissement et permettant de guider progressivement la partie supérieure (tête et cou) du volatile vers ce rétrécissement.

Le fond 13 du support 12 au niveau de ce rétrécissement présente une forme allongée définissant avec les rebords 14, un axe longitudinal 15.

L'opérateur qui a saisi un volatile en vue de sa vaccination, vient donc appuyer la partie supérieure du dos de cet animal contre le fond 13, ce dernier étant entouré par les rebords 14.

L'opérateur peut dès lors déplacer cet animal, tête la première, le long de cet axe longitudinal 15 vers une position d'injection du dispositif d'injection.

Le fond 13 du support 12 au niveau du rétrécissement comporte également un premier bouton d'actionnement 16, lequel est plan en s'étendant sur au moins une partie de ce fond. Ce bouton d'actionnement 16 est activé lorsque le cou du volatile, dans lequel l'injection doit être administrée, est plaqué contre le fond en étant reçu dans ce rétrécissement.

Ce rétrécissement est configuré pour assurer un calage en position du cou du volatile de sorte que son cou soit aligné ou sensiblement aligné le long de cet axe longitudinal 15 au niveau d'une zone d'injection définie par le déplacement de l'aiguille d'injection 21 du dispositif d'injection hors du corps principal 11.

Un second bouton d'actionnement 17 est placé sur une partie coudée définissant une butée 18 pour la tête du volatile.

Lorsque la partie supérieure du corps du volatile est appuyée contre ces deux boutons d'actionnement 16, 17 simultanément avec une force suffisante, un mécanisme d'entrainement (non représenté) placé dans le châssis 11 déplace l'aiguille d'injection 21, cette dernière passant d'une position de retrait, ou retractée, à l'intérieur du châssis 11, à une position d'injection extérieure au châssis 11.

Dans cette position d'injection, l'aiguille 21 se trouve placée dans le rétrécissement du support 12, de sorte qu'une injection peut être administrée dans le bas de la nuque du volatile. Cette nuque est avantageusement dégagée et calée en position lorsque le cou du volatile étant reçu dans ce rétrécissement, la tête du volatile est plaquée contre la partie coudée formant butée 18 pour la tête du volatile.

Entre ces positions de retrait et d'injection, l'aiguille est mobile en translation le long d'un axe de déplacement parallèle à la surface extérieure du fond du support, tout en étant parallèle à l'axe longitudinal 15.
Ainsi, la sortie de l'aiguille d'injection 21 se fait verticalement du haut vers le bas, en étant parallèle à la surface extérieure du fond 13 du support 12 de sorte que cette dernière ne peut aucunement pénétrer en profondeur dans le corps du volatile.

Au droit de la position d'injection, la distance séparant l'extrémité libre de l'aiguille d'injection 21 du fond 13 du support 12 est de 5 +/-2 mm de sorte qu'une injection sous cutanée de la substance liquide à administrer dans la nuque du volatile est ainsi garantie.

En outre, l'aiguille est excentrée par rapport à l'axe longitudinal 15 du support 12 de façon à ne pas injecter le produit au niveau de la colonne vertébrale de cet animal. On définit ainsi avantageusement une zone d'exclusion 22 entourant l'axe longitudinal 15 et dans laquelle l'aiguille d'injection 21 ne peut pénétrer et en conséquence, blesser l'animal.

Le chemin suivi par l'aiguille d'injection lors de son déplacement entre les positions de retrait et d'injection est de plus encadré par les rebords 14 du support 12. Ces rebords 14 forment avantageusement une barrière de protection pour l'opérateur contre toute blessure accidentelle.

D'une manière avantageuse, le fond 13 du support est légèrement incliné pour faciliter l'introduction de la partie supérieure du corps du volatile dans le rétrécissement.

L'extrémité inférieure du support 12 est en communication avec un toboggan 19 assurant une évacuation du volatile après administration de l'injection.

Le volatile vacciné peut dès lors être lâché par l'opérateur, le toboggan de forme incurvée amortissant sa chute. L'axe de chute du volatile et l'axe de déplacement de l'aiguille étant alignés, le risque de blessure du volatile est minimisé. Le volatile retombe sur ses pattes, car il reste toujours en position verticale, ou sensiblement verticale.

## Revendications

1. Dispositif d'injection d'un liquide dans un volatile, ledit dispositif d'injection comprenant un corps principal (11), lequel comporte un ensemble d'injection comprenant une aiguille d'injection et au moins une seringue, ladite aiguille étant mobile entre une position retractée et une position d'injection située dans une zone d'injection placée extérieurement audit corps principal (11), **caractérisé en ce qu'**il comprend :
- un élément de guidage (12) placé sur une surface extérieure du corps principal (11), ledit élément de guidage (12) comportant un fond (13) et des rebords latéraux (14) s'étendant à partir de ce fond, au moins la partie supérieure du dos de l'animal à vacciner étant destinée à être appuyée contre ce fond (13), et déplacée, tête la première, le long d'un axe de guidage (15) défini par ledit élément de guidage (12), vers ladite position d'injection,
- ledit élément de guidage (12) étant de plus configuré pour maintenir au moins le cou dudit volatile aligné ou sensiblement aligné le long de cet axe de guidage (15) au niveau de ladite zone d'injection,
- ladite aiguille d'injection étant agencée de sorte que lors de son déplacement entre lesdites positions retractée et d'injection, elle se déplace le long d'un axe de déplacement parallèle à la surface extérieure dudit fond, ou incliné par rapport à cette surface extérieure, cet axe de déplacement s'étendant, à l'extérieur dudit corps principal, hors d'une zone d'exclusion entourant ledit axe de guidage (15).

2. Dispositif d'injection selon la revendication 1, **caractérisé en ce que** l'axe de déplacement de ladite aiguille entre lesdites positions retractée et d'injection, est incliné d'un angle compris entre ]0, 4°] par rapport à la surface extérieure dudit fond (13).

3. Dispositif d'injection selon la revendication 1 ou 2, **caractérisé en ce que** ladite zone d'exclusion est un espace s'étendant sur 2 à 4 mm autour dudit axe de guidage (15).

4. Dispositif d'injection selon l'une quelconque des revendications 1 à 3, **caractérisé en ce que** l'axe de déplacement de ladite aiguille d'injection étant contenu dans un plan parallèle audit fond, ledit axe de déplacement et le dit axe de guidage (15) sont colinéaires ou ledit axe de déplacement diverge dudit axe de guidage (15).

5. Dispositif d'injection selon l'une quelconque des revendications précédentes, **caractérisé en ce que** ledit élément de guidage est configuré de sorte que la colonne vertébrale dudit volatile est placée dans ladite zone d'exclusion, lorsque au moins la partie supérieure du dos de l'animal à vacciner est plaquée contre ledit fond et que son cou est maintenu aligné ou sensiblement aligné le long de cet axe de guidage (15) au niveau de ladite zone d'injection.

6. Dispositif d'injection selon l'une quelconque des revendications précédentes, **caractérisé en ce que** au droit de la position d'injection, la distance séparant l'extrémité libre de ladite aiguille d'injection de la surface extérieure dudit fond (13) détermine une injection sous-cutanée dans le cou de ce volatile.

7. Dispositif d'injection selon l'une quelconque des revendications précédentes, **caractérisé en ce que** ce dispositif d'injection est configuré de sorte que son corps principal étant placé sur une surface horizontale plane, le volatile est injecté en position verticale, ou sensiblement verticale.

8. Dispositif d'injection selon l'une quelconque des revendications précédentes, **caractérisé en ce que** ledit élément de guidage (12) comporte une première partie de corps de forme évasée et une deuxième partie de corps formant un rétrécissement, ladite première partie de corps étant agencée pour assurer un guidage de la partie supérieure du corps du volatile vers ledit rétrécissement.

9. Dispositif d'injection selon l'une quelconque des revendications précédentes, **caractérisé en ce que** ledit corps principal (11) comporte des évidements (20) disposés latéralement à cet élément de guidage pour faciliter l'introduction par l'opérateur du volatile à injecter dans cet élément de guidage.

10. Dispositif d'injection selon l'une quelconque des revendications précédentes, **caractérisé en ce que** chaque ensemble d'injection comprenant un actionneur linéaire, ledit actionneur linéaire est un actionneur hydraulique, pneumatique ou électrique.

11. Dispositif d'injection selon l'une quelconque des revendications précédentes, **caractérisé en ce que** l'aiguille est déportée par rapport à la ou aux seringues.

12. Dispositif d'injection selon l'une quelconque des revendications précédentes, **caractérisé en ce qu'**il comporte deux seringues et une aiguille d'injection.

13. Dispositif d'injection selon l'une quelconque des revendications précédentes, **caractérisé en ce que** l'extrémité inférieure dudit élément de guidage (12) est en communication avec un toboggan (19) assurant une évacuation du volatile après administration de l'injection.

14. Dispositif d'injection selon l'une quelconque des revendications précédentes, **caractérisé en ce que** les angles des pièces du dispositif avec lesquelles le volatile à injecter et l'opérateur sont susceptibles d'entrer en contact sont arrondis.

15. Dispositif d'injection selon l'une quelconque des revendications précédentes, **caractérisé en ce que** l'extrémité supérieure dudit corps principal présente un coude pour imposer une inclinaison particulière à la tête du volatile, ce coude comportant un ou plusieurs éléments de maintien configurés pour bloquer en position la tête du volatile à vacciner.

## Patentansprüche

1. Vorrichtung zum Injizieren einer Flüssigkeit in einen Vogel, wobei die Injektionsvorrichtung einen Hauptkörper (11) umfasst, der eine eine Injektionsnadel und mindestens eine Spritze umfassend Injektionsanordnung aufweist, wobei die Nadel zwischen einer zurückgezogenen Position und einer Injektionsposition, die sich in einem außerhalb des Hauptkörpers (11) liegenden Injektionsbereich befindet, beweglich ist, **dadurch gekennzeichnet, dass** sie Folgendes umfasst:
- ein Führungselement (12), das an einer Außenfläche des Hauptkörpers (11) platziert ist, wobei das Führungselement (12) einen Boden (13) und seitliche Ränder (14), die sich von diesem Boden aus erstrecken, aufweist, wobei zumindest für den oberen Teil des Rückens des zu impfenden Tieres vorgesehen ist, dass dieser an diesen Boden (13) gedrückt wird und mit dem Kopf voran entlang einer durch das Führungselement (12) definierten Führungsachse (15) in Richtung der Injektionsposition bewegt wird,
- wobei das Führungselement (12) ferner konfiguriert ist, um mindestens den Hals des Vogels entlang dieser Führungsachse (15) im Bereich des Injektionsbereichs ausgerichtet oder im Wesentlichen ausgerichtet zu halten,
- wobei die Injektionsnadel so angeordnet ist, dass sie sich bei ihrer Bewegung zwischen der zurückgezogenen Position und der Injektionsposition entlang einer Bewegungsachse bewegt, die parallel zur Außenfläche des Bodens oder geneigt zu dieser Außenfläche verläuft, wobei sich diese Bewegungsachse außerhalb des Hauptkörpers aus einem die Führungsachse (15) umgebenden Ausschlussbereich heraus erstreckt.

2. Injektionsvorrichtung nach Anspruch 1, **dadurch gekennzeichnet, dass** die Bewegungsachse der Nadel zwischen der zurückgezogenen Position und der Injektionsposition in einem Winkel zwischen ]0, 4°] zur Außenfläche des Bodens (13) geneigt ist.

3. Injektionsvorrichtung nach Anspruch 1 oder 2,
**dadurch gekennzeichnet, dass** der Ausschlussbereich ein Bereich ist, der sich 2 bis 4 mm um die Führungsachse (15) erstreckt.

4. Injektionsvorrichtung nach einem der Ansprüche 1 bis 3,
**dadurch gekennzeichnet, dass** die Bewegungsachse der Injektionsnadel in einer zum Boden parallelen Ebene liegt, wobei die Bewegungsachse und die Führungsachse (15) kolinear sind oder die Bewegungsachse von der Führungsachse (15) abweicht.

5. Injektionsvorrichtung nach einem der vorstehenden Ansprüche,
**dadurch gekennzeichnet, dass** das Führungselement so konfiguriert ist, dass die Wirbelsäule des Vogels in dem Ausschlussbereich platziert ist, wenn mindestens der obere Teil des Rückens des zu impfenden Tieres gegen den Boden gedrückt wird und sein Hals entlang dieser Führungsachse (15) im Injektionsbereich ausgerichtet oder im Wesentlichen ausgerichtet gehalten wird.

6. Injektionsvorrichtung nach einem der vorstehenden Ansprüche, **dadurch gekennzeichnet, dass** an der Stelle der Injektionsposition der Abstand zwischen dem freien Ende der Injektionsnadel und der Außenfläche des Bodens (13) eine subkutane Injektion in den Hals des Vogels festlegt.

7. Injektionsvorrichtung nach einem der vorstehenden Ansprüche,
**dadurch gekennzeichnet, dass** diese Injektionsvorrichtung so konfiguriert ist, dass, wenn ihr Hauptkörper auf einer horizontalen ebenen Fläche platziert wird, der Vogel in vertikaler oder im Wesentlichen vertikaler Position geimpft wird.

8. Injektionsvorrichtung nach einem der vorstehenden Ansprüche,
**dadurch gekennzeichnet, dass** das Führungselement (12) einen ersten, sich erweiternden Körperabschnitt und einen zweiten, eine Verengung bildenden Körperabschnitt aufweist, wobei der erste Körperabschnitt so angeordnet ist, dass er ein Führen des oberen Teils des Körpers des Vogels in Richtung der Verengung sicherstellt.

9. Injektionsvorrichtung nach einem der vorstehenden Ansprüche, **dadurch gekennzeichnet, dass** der Hauptkörper (11) seitlich zu diesem Führungselement angeordnete Aussparungen (20) aufweist, um dem Verabreichenden das Einführen des zu impfenden Vogels in dieses Führungselement zu erleichtern.

10. Injektionsvorrichtung nach einem der vorstehenden Ansprüche, **dadurch gekennzeichnet, dass** jede Injektionsanordnung einen Linearaktuator umfasst, wobei der Linearaktuator ein hydraulischer, pneumatischer oder elektrischer Aktuator ist.

11. Injektionsvorrichtung nach einem der vorstehenden Ansprüche, **dadurch gekennzeichnet, dass** die Nadel gegenüber der oder den Spritzen versetzt angeordnet ist.

12. Injektionsvorrichtung nach einem der vorstehenden Ansprüche, **gekennzeichnet dadurch, dass** sie zwei Spritzen und eine Injektionsnadel aufweist.

13. Injektionsvorrichtung nach einem der vorstehenden Ansprüche, **dadurch gekennzeichnet, dass** das untere Ende des Führungselements (12) mit einer Gleitbahn (19) in Verbindung steht, die nach dem Verabreichen der Injektion das Austragen des Vogels sicherstellt.

14. Injektionsvorrichtung nach einem der vorstehenden Ansprüche, **dadurch gekennzeichnet, dass** die Winkel der Teile der Vorrichtung, mit denen der zu impfende Vogel und der Verabreichende in Kontakt kommen können, abgerundet sind.

15. Injektionsvorrichtung nach einem der vorstehenden Ansprüche, **dadurch gekennzeichnet, dass** das obere Ende des Hauptkörpers eine Biegung aufweist, um dem Kopf des Vogels eine bestimmte Neigung aufzuzwingen, wobei diese Biegung ein oder mehrere Halteelemente umfasst, die so konfiguriert sind, dass sie den Kopf des zu impfenden Vogels in seiner Position fixieren.

## Claims

1. Device for injecting a fluid into a bird, said injection device comprising a main body (11) which has an injection assembly comprising an injection needle and at least one syringe, said needle being movable between a retracted position and an injecting position located in an injection zone positioned outside said main body (11), **characterized in that** it comprises:
- a guide element (12) positioned on an exterior surface of the main body (11), said guide element (12) having a base (13) and side edges (14) extending from this base, at least the upper part of the back of the animal to be vaccinated being intended to rest against this base (13) and to be moved toward said injecting position headfirst along a guide axis (15) defined by said guide element (12),
- said guide element (12) being additionally configured to keep at least the neck of said bird aligned or substantially aligned along this guide axis (15) in the injection zone,
- said injection needle being arranged such that, during its movement between said retracted and injecting positions, it moves along a movement axis parallel to the exterior surface of said base or inclined relative to this exterior surface, this movement axis extending, outside the main body, beyond an exclusion zone surrounding said guide axis (15).

2. Injection device according to claim 1, **characterized in that** the movement axis of said needle between said retracted and injecting positions is inclined by an angle of between [0, 4°] relative to the exterior surface of said base (13).

3. Injection device according to claim 1 or 2, **characterized in that** said exclusion zone is a space extending over 2 to 4 mm about said guide axis (15).

4. Injection device according to any one of claims 1 to 3,
**characterized in that** the movement axis of said injection needle is contained in a plane parallel to said base, said movement axis and said guide axis (15) being collinear or said movement axis diverging from said guide axis (15).

5. Injection device according to any one of the preceding claims, **characterized in that** said guide element is configured such that the spine of said bird is positioned in said exclusion zone when at least the upper part of the back of the animal to be vaccinated is pressed against said base and its neck is kept aligned or substantially aligned along this guide axis (15) in said injection zone.

6. Injection device according to any one of the preceding claims, **characterized in that** in line with the injecting position, the distance separating the free end of said injection needle from the exterior surface of said base (13) determines a subcutaneous injection into the neck of this bird.

7. Injection device according to any one of the preceding claims, **characterized in that** this injection device is configured such that when its main body is positioned on a flat, horizontal surface, the bird is injected in the vertical or substantially vertical position.

8. Injection device according to any one of the preceding claims, **characterized in that** said guide element (12) has a first body part that is flared and a second body part forming a narrowing, said first body part being arranged to guide the upper part of the body of the bird toward said narrowing.

9. Injection device according to any one of the preceding claims, **characterized in that** said main body (11) has recesses (20) disposed laterally with respect to this guide element in order to facilitate the insertion of the bird to be injected into this guide element by the operator.

10. Injection device according to any one of the preceding claims, **characterized in that** each injection assembly comprises a linear actuator, said linear actuator being a hydraulic, pneumatic or electric actuator.

11. Injection device according to any one of the preceding claims, **characterized in that** the needle is offset relative to the syringe or syringes.

12. Injection device according to any one of the preceding claims, **characterized in that** it has two syringes and one injection needle.

13. Injection device according to any one of the preceding claims, **characterized in that** the lower end of said guide element (12) is in communication with a slide (19) discharging the bird after the injection has been administered.

14. Injection device according to any one of the preceding claims, **characterized in that** the corners of the parts of the device with which the bird to be injected and the operator are able to come into contact are rounded.

15. Injection device according to any one of the preceding claims, **characterized in that** the upper end of said main body has a bend to impose a particular inclination on the head of the bird, this bend having one or more holding elements configured to keep the head of the bird to be vaccinated in position.
